Europäisches Patentamt

European Patent Office

Office européen des brevets

(11) Publication number: **0 361 912**
**A1**

## EUROPEAN PATENT APPLICATION

(21) Application number: **89309873.1**

(22) Date of filing: **28.09.89**

(51) Int. Cl.5: **A61L 27/00**

(30) Priority: **28.09.88 US 250373**

(43) Date of publication of application:
**04.04.90 Bulletin 90/14**

(84) Designated Contracting States:
**CH DE FR GB LI NL**

(71) Applicant: **Regan, Barrie F.**
**2260 Redington Road**
**Hillsborough California 94010(US)**

(72) Inventor: **Regan, Barrie F.**
**2260 Redington Road**
**Hillsborough California 94010(US)**

(74) Representative: **Bowman, Paul Alan et al**
**LLOYD WISE, TREGEAR & CO. Norman**
**House 105-109 Strand**
**London WC2R OAE(GB)**

(54) **Non-thrombotic metal prostheses.**

(57) Non-thrombotic prostheses comprising a corrosion resistant metal and a metallic tin coating on the surface of the corrosion resistant metal.

EP 0 361 912 A1

## NON-THROMBOTIC METAL PROSTHESES

Development of the balloon angioplasty technique began about ten years ago. The purpose of this technique is to open arteries in which the flow of blood has been impeded by build-up of arteriosclerotic plaques on the interior walls of the arteries. The technique consists in inserting a small diameter catheter into a blocked artery, the catheter has a small flexible balloon attached at its distal end. The catheter is moved thru the artery until the balloon is placed in the area of the artery in which blood flow is impeded by plaque build-up. The balloon is then inflated and flattens the plaque against the artery walls opening the artery to permit improved blood flow. the balloon is then deflated and removed leaving the plaque flattened against the artery walls.

After passage of several months time a proportion of the treated arteries, approximating one third, undergo restenosis, a reclosing of the artery at the treated area, requiring repetition of balloon angioplasty. The restenosis problem has received considerable attention and proposals have been made to deal with it. the most promising approach to restenosis prevention is the placement of a stent in the blood vessel which has undergone balloon angioplasty at the position in the vessel where the balloon was inflated immediately following removal of the balloon. The term stent is now in common use to denote a short tube, open at both ends for insertion in a blood vessel following balloon angioplasty to prevent restenosis, terms other than stent such as graft prosthesis, arterial endoprothesis, intraluminal graft and intravascular mechanical support may be and are frequently used instead of "stent" to convey the same meaning.

This application relates to improved prostheses and is a continuation-in-part of application Serial No. 069,364 filed July 2, 1987, now U.S. Patent No. 4,795,458.

Dotter patent 4,503,569 for Transluminally Placed Expandable Graft Prosthesis describes a stent in detail. A Dotter et al. paper titled Transluminally Expendable Coil Stent Grafting was published in Radiology 147:259-60 a month after his patent application was filed. Paper and patent are directed to the same subject matter. It is of passing interest that a Cragg et al. paper titled Nonsurgical Placement of Arterial Endoprotheses: A New Technique Using Nitinol Wire was published in Radiology 147:261-263 and is essentially identical in content with the Dotter et al. paper.

Both papers and the patent describe the making of a stent from Nitinol, a shape memory alloy of titanium and nickel. The stent is a short helical coil of Nitinol wire. The diameter of the helical coil is equal to or slightly greater than that of the blood vessel in which it is intended to use the stent. After the helical coil is made it is heated to fix the shape of the coil in the memory of the Nitinol. The wire of the helical coil is then wound to form a helical coil having an appreciably smaller diameter than that of the first helical coil. The smaller diameter coil is then placed in the blood vessel at the place where the balloon was inflated. After placement in the blood vessel the coil is heated by passing warm (115-125°F) saline solution thru a catheter to heat the stent. Upon being heated the metal of the stent returns to its first larger diameter shape and presses firmly against the blood vessel walls where it is left to hold the blood vessel open preventing restenosis.

Both Dotter et al. and Cragg et al., in their papers expressed the opinion that Nitinol in addition to its shape memory which facilitated fixing a Nitinol stent at a position in the blood vessel was resistant to formation of thrombi on its surface. Cragg et al. summarized their view saying, "By using Nitinol wire, the two main problems associated with this technique, namely, thrombosis of the endoprosthesis and difficulty in introducing a graft of suitable size through conventional angiographic catheters, appear to be solved."

Since the publication of the Cragg et al. and Dotter et al. papers in April 1983 further experience with Nitinol prostheses has shown that thrombus formation does occur on the surface of Nitinol stents planted in the arteries. ( Wright et al. Radiology 1985; 156:69-72)

The ease with which a Nitinol stent can be fixed at a desired position in a blood vessel was and is indeed a valuable property. Work was therefore undertaken with a view to reducing or eliminating thrombus formation on the surface of the Nitinol.

It was observed that Nitinol has a potential of +0.4 volts in the electromotive series of metals, a potential much higher than human body potential. It was conceived that if the Nitinol potential could be brought to a near-body level thrombosis might be reduced or prevented. Pursuit of line of thought led to the idea of covering the Nitinol surface with a thin layer of tin which has a potential of +0.14 volt. The tin forming the covering layer may contain 1 to 10 percent by weight of indium.

The tin coating may be laid down on the alloy by any conventional method, electroplating, sputtering, vacuum evaporation and the like. The tin coating may be covered with a very thin coating of indium about 100 to 1000 Angstrom units in thickness preferably by electroplating and the stent is then heated to a temperature near the indium melting point for example to a temperature of 150° C for a time sufficient to cause diffusion of indium into the tin coating increasing its resistance to corrosion. The indium content of the tin coating after diffusion of the indium into the tin layer is about 1% to 10% by weight.

The prosthesis illustrated in the drawing is a stent for implantation in a blood vessel following balloon angioplasty. A stent is defined at page 1 of the specification as follows:

"The term stent is now in common use to denote a short tube open at both ends for insertion in a blood vessel following balloon angioplasty to prevent restenosis."

The stent may be formed from corrosion resistant metal or metal alloy such as Nitinol. The surface of the corrosion resistant metal or alloy is covered with a thin coating of tin. The thin coating of tin renders the stent non-thrombotic.

Figure 1 of the drawings shows the tubular stent 1 formed from corrosion resistant metal 2. A thin coating of tin 3 covers the surface of the corrosion resistant metal from which the stent is formed.

Figure 2 of the drawings is a cross section of the stent showing the corrosion resistant metal 2 forming the tube and a thin tin coating covering the surface of the corrosion resistant metal.

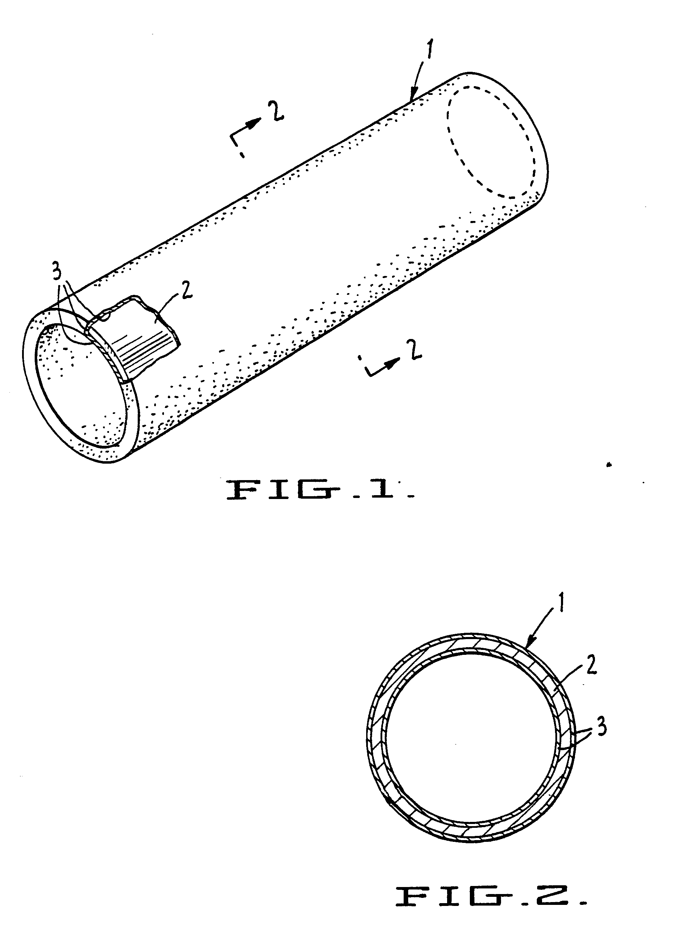

Stents of these kinds have been implanted in veins or arteries of a number of human patients who have undergone balloon angioplasty. Some of the patients had experienced restenosis of a blood vessel following balloon angioplasty and required a second balloon angioplasty which was immediately followed by implantation of the stent. The remainder of the patients had undergone balloon angioplasty which was immediately followed by implantation of the stent.

All of the patients have been regularly checked for evidence of thrombus formation on the stent surfaces. Checks were made by x-ray, pressure gradient measurement and Doppler evaluation. No evidence whatever of thrombus formation has been found in any patient. Most of the patients have been regularly checked for evidence of thrombus formation for more than a year and no evidence has been found.

The work on tin coated Nitinol stents made it clear that when Nitinol, a thrombotic metal, was covered with a tin coating the only metal contacted by the blood of the patient was the tin coating. No thrombi formed on the tin surface.

A variety of prosthetic devices made from corrosion resistant metals have been put to use in the cardiovascular systems of human beings. Prosthetic heart valves, pumps and filters are in use.

The formation of thrombi on the metal surfaces of these prostheses is a common and continuing problem associated with the use of these devices. This problem can be alleviated or eliminated by covering the corrosion resistant metal surface with a thin coating of metallic tin. The tin coating may be placed on the surface of the corrosion resistant metal by methods such as sputtering, vacuum evaporation or electroplating.

Electroplating is the preferred method for applying the coating since this method will produce a coating of predetermined and uniform thickness. A suitable plating bath and plating conditions are:

Plating bath

225-300 g/L Stannous fluoborate
225-300 g/L fluoboric acid
22.5-37.5 g/L boric acid

Conditions

Anode - pure tin
Cathode - corrosion resistant metal to be plated
Cathode current density ASF - up to 300
Anode current density ASF - not above 25

When the corrosion resistant metal which forms the body of the prosthesis is connected to the cathode and the flow of current is initiated, metallic tin is deposited on the corrosion resistant metal. Passage of current is continued until a tin coating of desired thickness is built up on the surface of the corrosion resistant metal. Generally the coating thickness need only be 0.0001 to 0.0002 inches but coating of greater thickness e.g. 0.001 inch maybe used if desired. When the prosthesis is implanted in the cardiovascular system of a human being the blood contacts only the tin coating on the surface of the corrosion resistant metal and thrombi are not formed on the tin surface. suitable corrosion resistant metals for forming the body of the prosthesis include stainless steel silver, platinum, tantalum, vanadium, chromium, gold, tungsten and molybdenum. In general, however, metals which are oxidation resistant and not attacked by body fluids may be used to form the body of the prosthesis. Stainless steels are much used in shaping the bodies of various prostheses and it is found that the tendency of stainless steels to thrombose is effectively suppressed by coating the stainless steel surface with tin.

Use of prostheses as described herein avoids the problems associated with thrombus formation on the prostheses surfaces.

## Claims

1. A non-thrombotic metal prosthesis for use in the cardiovascular system of a human being, comprising a corrosion resistant metal substrate sized and shaped to fit into the site of the cardiovascular system requiring implantation of the prosthesis and a thin coating of tin covering the surface of the substrate.

2. The prosthesis of Claim 1 wherein the tin coating contains from about one to ten percent by weight of indium.

3. The prosthesis of Claim 1 wherein the corrosion resistant metal substrate is stainless steel.

FIG.1.

FIG.2.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,P X | US-A-4 795 458  (B.F. REGAN) <br> * Whole document * <br> --- | 1-3 | A 61 L  27/00 |
| A | FR-A-2 277 792  (NATIONAL RESEARCH) <br> ----- | | |

TECHNICAL FIELDS SEARCHED (Int. Cl.5)

A 61 L

The present search report has been drawn up for all claims

| Place of search | Date of completion of the search | Examiner |
|---|---|---|
| THE HAGUE | 12-12-1989 | PELTRE CHR. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)